# EUROPEAN PATENT SPECIFICATION

(11) **EP 4 133 279 B1**
(45) Date of publication and mention of the grant of the patent: **24.01.2024**
(21) Application number: 21710020.5
(22) Date of filing: 10.02.2021
(51) Int. Cl.: G01N 33/68, G01N 33/53

(54) **A METHOD FOR DETERMINING A LECTIN-BINDING GLYCAN INDICATIVE TO TRAUMATIC BRAIN INJURY**
VERFAHREN ZUR BESTIMMUNG EINES LEKTINBINDENDEN GLYCANS ZUR ANZEIGE TRAUMATISCHER HIRNVERLETZUNGEN
MÉTHODE DE DÉTERMINATION D'UN GLYCANE SE LIANT AUX LECTINES INDIQUANT UN TRAUMATISME CRANIOCÉRÉBRAL

(30) Priority: 06.04.2020 US 202016840931
(43) Date of publication of application: 15.02.2023
(73) Proprietor: Medicortex Finland Oy, 20520 Turku (FI)
(72) Inventor: HAREL, Adrian, 20520 Turku (FI); KVIST, Mårten, 20520 Turku (FI); VÄLIMAA, Lasse, 20520 Turku (FI); UTZ, Begüm, 20520 Turku (FI); HAAVISTO, Oskar, 20520 Turku (FI); NURMI, Venla-Mari, 20520 Turku (FI)
(74) Representative: Hovinen, Jari Juhani
(86) International application number: PCT/FI2021/050091
(87) International publication number: WO 2021/205059

(56) References cited:
- WO-A1-2010/102285
- WO-A1-2014/133428
- WO-A1-2018/154401
- WO-A1-2021/099677
- GB-A- 2 404 734
- ABOU-ABBASS HUSSEIN ET AL: "Deciphering glycomics and neuroproteomic alterations in experimental traumatic brain injury: Comparative analysis of aspirin and clopidogrel treatment : Proteomics and 2-DE", ELECTROPHORESIS, vol. 37, no. 11, 1 June 2016 (2016-06-01), pages 1562-1576, XP055821264, ISSN: 0173-0835, DOI: 10.1002/elps.201500583
- BERTOK TOMAS ET AL: "Label-free detection of glycoproteins by the lectin biosensor down to attomolar level using gold nanoparticles", TALANTA, ELSEVIER, AMSTERDAM, NL, vol. 108, 1 March 2013 (2013-03-01), pages 11-18, XP028579772, ISSN: 0039-9140, DOI: 10.1016/J.TALANTA.2013.02.052

## Description

### FIELD

The present invention relates to a method for determining a lectin-binding glycan indicative to traumatic brain injury (TBI), in particular to methods wherein the determining is based on detection of the lectin-binding glycan in a fluid sample using lectins of similar glycan binding property for tracing and capturing.

### BACKGROUND

Traumatic brain injury (TBI) is the leading cause of central nervous system impairment in these days, with more than 2.5 million individuals suffering annually from TBI in the US alone. According to the CDC, the highest incidence of TBI occurs among children 0-4 years old, adolescents 15-19 years old, and adults over 65 years of age. Despite the broad range of the population affected, TBI is still under-served and remains an unexplored pathological condition.

Traditionally, TBI has been acutely diagnosed and classified by neurological examinations, such as Glasgow Coma Scale (GCS). However, the use of the GCS as a diagnostic tool is subject to a number of limitations. Recent research has provided evidence that the use of sedative drugs precludes accurate GCS assessment during the first 24 hours. Further challenges to diagnosis are presented by the evolving nature of some brain lesions, which can lead to further neurological impairment. In addition, neurological responses after TBI can vary over time for reasons unrelated to the injury. Still further challenges include the trauma subject's possible unconsciousness or inability to communicate.

Neuroimaging techniques, such as x-ray, CT scanning and MRI, are used to provide information on injury magnitude and location and are not influenced by the aforementioned disadvantages. However, CT scanning has low sensitivity to diffuse brain damage, and availability and utility of MRI is limited. MRI is also very impractical to perform if subjects are physiologically unstable and can lead to inaccurate diagnoses in military injuries in which metal fragments in the body are common.

Mild and moderate TBI represent more than 90 % of TBI injuries; this injury range represents the greatest challenges to accurate acute diagnosis and outcome prediction. Unlike severe TBI, there is no universally recognized neurologic assessment scale such as the GCS, and many cases of mild TBI are classified as subclinical brain injury (SCI). The widespread recognition of inadequate approaches to diagnose mild TBI indicate the need for significant improvement in the diagnosis and classification of TBI, such as the use of biomarkers to supplement functional and imaging-based assessments. These biomarkers can be altered gene expression, protein, lipid or glycan metabolites, or a combination of these changes after a traumatic brain injury, reflecting the initial insult (the primary injury) and the evolution of a cascade of secondary damage (the secondary injury). In particular, subclinical brain injury status or SCI could be diagnosed with a biomarker analysis.

As with many injuries, increased serum levels of cytokines and chemokines have been noted post-TBI and, as such, have been proposed as potential surrogate markers for TBI outcome. However, to date, there are not too many biomarkers for the diagnosis or prognosis of TBI. This is because of several obstacles to the development of reliable blood biomarkers of TBI. For instance, the blood-brain barrier (BBB) hinders the assessment of biochemical changes in the brain by use of blood biomarkers in mild TBI, although impaired BBB integrity, as seen in severe TBI, can increase the levels of brain-derived proteins in the blood. Nevertheless, owing to their dilution in the much larger plasma volume, biomarkers that are highly expressed within the central nervous system exist at very low concentrations in blood. Moreover, some potential biomarkers undergo proteolytic degradation in the blood, and their levels might be affected by clearance from blood via the liver or kidney. As a consequence, reliable blood biomarkers have been extremely difficult to identify.

WO/2016/166419 by the present assignee and one of the present inventors, discloses glycan-based biomarkers for the diagnosis and prognosis of brain damage, such as traumatic brain injury (TBI), subclinical brain injury (SCI) and acquired brain injury (ABI). The glycan-based biomarker protocol disclosed therein may be used as an end point in clinical trials and in other diagnostic tests to determine, qualify, and/or assess brain injury status, for example, to diagnose brain injury, in an individual, subject, or patient. As part of the diagnosis afforded by the glycan-based biomarker disclosed therein, brain injury status can include determination of a subject's subclinical brain injury status or SCI status, for example, to diagnose SCI, in an individual, subject, or patient.

WO/2018/154401 by the present assignee and the present inventors discloses a device for conducting a non-invasive analysis of a bodily fluid, such as saliva or urine, to determine the presence and the level of a certain glycan-based biomarkers that are indicative of brain injury, that are carried by the bodily fluid. The device includes an indicator formulation capable of changing color in response to exposure to the biomarkers to provide a visual indication of the presence and the level of the biomarkers carried by the bodily fluid. The device comprises a porous matrix substrate for establishing a high void volume within the carrier substrate, and an indicator formulation carried by the carrier substrate. The indicator formulation includes a chromogen agent (a visually detectable label) and a biomarker-specific agent selected from a variety of agents responsive to levels of any one of a plurality of different glycan-based biomarkers that are indicative of brain injury.

Historic obstacles to point-of-care devices include manufacturing challenges, ease-of-use limitations, and government regulations. Some of these obstacles have been reduced through advances in technology and recognition by governments and other regulatory bodies of the importance of point-of-care testing. However, important considerations, including ease-of-use and accuracy, still render point-of-care tests unsuitable for many healthcare facilities and domestic settings, and more so for particular medical conditions, such as brain injury.

One challenge is that the biomarkers indicative to TBI are often present also in body fluid of subjects not suffering from TBI. Thus, the presence of the marker in body fluid may not be an indication of TBI. Accordingly, there is still need for further methods for determining brain injury.

### SUMMARY

The present invention is based on the observation that when similar lectins are used for tracing and capturing a lectin-binding glycan indicative to traumatic brain injury, some of the prior art problems can be avoided or at least alleviated.

Accordingly, it is an object of the present invention to provide a method for determining a lectin-binding glycan indicative to traumatic brain injury (TBI) in a fluid sample the method comprising
a) providing
   i. a probe comprising
      ∘ a porous matrix, the porous matrix comprising a first part, a second part and a detection zone therebetween and
      ∘ a first lectin immobilized on the detection zone, the first lectin adapted to bind to the lectin-binding glycan,
   ii. a conjugate comprising
      ∘ a second lectin adapted to bind to the lectin-binding glycan and
      ∘ a signal generation means adapted to produce a detectable signal upon binding of the second lectin to the lectin-binding glycan immobilized to the detection zone via the first lectin,
      wherein the first lectin and the second lectin are same lectins and are selected from a group consisting of Urtica dioica (UDA), Allium savitum (ASA) and Phytohemagglutinin-L (PHA-L),
b) exposing the detection zone to the fluid sample,
c) exposing the detection zone to the conjugate,
d) detecting signal derived from the signal generation means on the detection zone,
e) determining level of the lectin-binding glycan in the fluid sample based on the detecting, and
f) comparing the level of the lectin-binding glycan in the fluid sample to a control level indicative to a level of the lectin-binding glycan in a body fluid of a subject not suffering from TBI, wherein said lectin-binding glucan is indicative to TBI when its level in the fluid sample is at least two times higher than the control level.

It is also an object of the present invention to provide a method for determining traumatic brain injury (TBI) in a subject, the method comprising
a) providing
   i. a probe comprising a porous matrix, the porous matrix comprising
      ∘ a first part, a second part and a detection zone therebetween and
      ∘ a first lectin immobilized to the detection zone, the first lectin adapted to bind to a lectin-binding glycan indicative to traumatic brain injury and
   ii. a conjugate comprising
      ∘ a second lectin adapted to bind to the lectin-binding glycan indicative to traumatic brain injury and
      ∘ a signal generation means adapted to produce a detectable signal upon binding of the second lectin to the lectin-binding glycan immobilized to the detection zone via the first lectin,
      wherein the first lectin and the second lectin are same lectins and are selected from a group consisting of Urtica dioica (UDA), Allium savitum (ASA) and Phytohemagglutinin-L (PHA-L),
b) exposing the detection zone to a body fluid sample obtained from the subject,
c) exposing the detection zone to the conjugate,
d) detecting signal derived from the signal generation means at the detection zone,
e) determining level of the lectin-binding glycan in the body fluid sample based on the detecting,
f) comparing the level of the lectin-binding glycan in the sample to a control level indicative to a level of the lectin-binding glycan in body fluid of a subject not suffering from TBI, wherein said lectin-biding glycan is indicative to TBI when its level in the fluid sample is at least two times higher than the control level and
g) determining TBI in the subject based on the comparing, wherein elevated level is an indication of TBI in the subject.
Further objects of the present invention are described in the accompanying dependent claims.

Exemplifying and non-limiting embodiments of the invention, both as to constructions and to methods of operation, together with additional objects and advantages thereof, are best understood from the following description of specific exemplifying embodiments when read in connection with the accompanying drawings.

The verbs "to comprise" and "to include" are used in this document as open limitations that neither exclude nor require the existence of un-recited features. The features recited in the accompanied depending claims are mutually freely combinable unless otherwise explicitly stated. Furthermore, it is to be understood that the use of "a" or "an", i.e. a singular form, throughout this document does not exclude a plurality.

### BRIEF DESRCIPTION OF THE DRAWINGS

Figure 1 illustrates a workflow of a lectin sandwich assay protocol according to an exemplary non-limiting embodiment of the present invention,
Figure 2 illustrates an exemplary test strip for determining increased level or normal level of a lectin-binding glycan in a fluid sample,
figure 3 illustrates a flowchart of a method according to an exemplary non-limiting embodiment of the present invention for determining the presence or absence of TBI in a subject,
figure 4 illustrates an exemplary test strip for determining the presence or absence of traumatic brain injury in a subject, and
figure 5 illustrates results from lectin capture-tracer pair comparison quantitated with *ImageJ* software (TBI = sample from a subject suffering from traumatic brain injury; HC = healthy control sample from a subject not suffering from traumatic brain injury).
Figure 6 illustrates probes i.e., test strips for determining traumatic brain injury in a subject, first part (1); detection zone (2); second part (3); position of the first lectin on the detection zone (2a). The arrow shows the flow direction.
Figure 7 illustrates the pixel intensity of the spots in the detection zone (lectin/glycan-binding areas) of figure 6 quantitated using ImageJ software.
Figure 8 illustrates probes i.e., test strips for determining traumatic brain injury in a subject, first part (1); detection zone (2); position of the first lectin on the detection zone (2a). The arrow shows the flow direction. Second part of the test strip is not shown in the figure.
Figure 9 illustrates probes i.e., test strips for determining traumatic brain injury in a subject, first part (1); detection zone (2); second part (3); position of the first lectin on the detection zone (2a). The arrow shows the flow direction.

### DESCRIPTION

According to one embodiment, the present invention concerns a method for determining a lectin-binding glycan indicative to traumatic brain injury (TBI) in a fluid sample. The method comprises the following steps:
a) providing
   i. a probe comprising a porous matrix, the porous matrix comprising
      ∘ a first part, a second part and a detection zone therebetween and
      ∘ a first lectin immobilized to the detection zone, and
   ii. a conjugate comprising
      ∘ a second lectin and
      ∘ a signal generation means adapted to produce a detectable signal upon binding of the second lectin to the lectin-binding glycan on the detection zone via the first lectin
      wherein the first lectin and the second lectin have similar glycan-binding properties,
b) exposing the detection zone to the fluid sample,
c) exposing the detection zone to the conjugate,
d) detecting signal derived from the signal generation means at the detection zone, and
e) determining level of the lectin-binding glycan in the fluid sample based on the detecting.

As defined herein a probe is a device to obtain information for diagnostic purposes. Exemplary probes are a test strip and a glass slide.

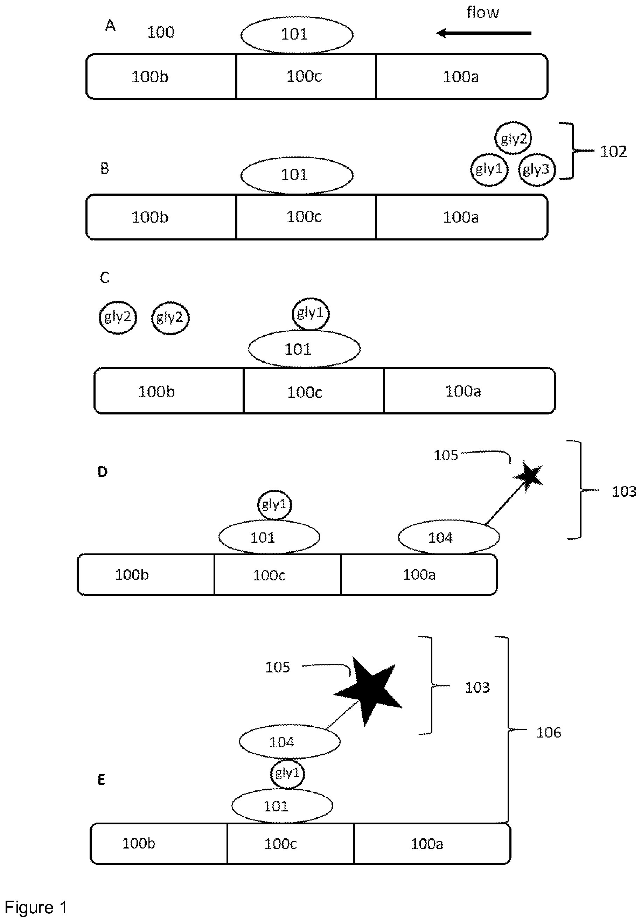

An exemplary non-limiting workflow of the method is shown in figure 1. The arrow in the figure shows the flow direction.

Figure 1A shows a probe comprising a porous matrix 100. The porous matrix comprises a first part 100a, a second part 100b and a detection zone 100c therebetween. A first lectin 101 is immobilized on the detection zone. The first lectin is adapted to bind the lectin-binding glycan indicative to TBI. The binding is selective.

Figure 1B shows a situation where a fluid sample 102 comprising three lectin-binding glycans (gly1, gly2, gly3) has been applied on the first part of the porous matrix. The sample is allowed to elute from the first part of the matrix towards the second part through the detection zone. The aim is to expose the detection zone comprising the immobilized first lectin to glycans of the fluid sample.

Figure 1C shows a situation where one of the glycans (gly1) has bound to the immobilized lectin 101. Other glycans do not bind to the immobilized lectin but elute through the detection zone to the second part. Removal of the unbound glycans and other material present in the sample can be performed by e.g. by applying a wash solution to the first part of the porous matrix and allowing the wash solution to elute through the detection zone towards the second part, i.e. to flush the matrix. According to another exemplary embodiment the removal of the unbound glycans and other material is performed by soaking the probe to a wash solution. An exemplary was solution is saline such as PBS.

Figure 1D shows a situation where the unbound glycans (gly2 and gly3) have been washed away, and a conjugate 103, or a solution comprising the conjugate comprising a second lectin 104 and a signal generation means 105 has been applied on the first part of the porous matrix. The second lectin is adapted to bind the lectin-binding glycan. The binding is selective.

The immobilized lectin, i.e., the first lectin, and the second lectin have similar glycan-binding properties. According to a particular embodiment the lectins are similar lectins, preferably same lectins. The conjugate is adapted to elute from the first part towards the second part through the detection zone.

Figure 1E shows a situation where the conjugate has eluted from the first part to the detection zone. The second lectin 104 of the conjugate binds to the glycan (gly1) immobilized to the detection zone via the first lectin 101. Removal of the unbound glycans and other material present in the sample can be performed by e.g. by applying a wash solution to the first part of the porous matrix and allowing the wash solution to elute through the detection zone towards the second part, i.e. to flush the matrix. The binding causes increase of concentration of signal generating means at the detection zone. The level of the signal is dependent on the amount of the lectin-binding glycan (gly1) in the sample.

### The fluid sample

The fluid sample can be any sample expected to include glycans, in particular lectin-binding glycans indicative to TBI. Particular lectin-binding glycans comprise sequences listed in table 1.

A particular fluid is body fluid. The fluid sample is collected from a TBI patient preferably by non-invasive or minimally invasive methods, or, alternatively, by invasive methods. Exemplary body fluids are saliva, urine, serum, blood plasma, tears, cerebrospinal fluid, oral secretions, respiratory secretions. A particular body fluid is saliva. Another particular body fluid is urine.

According to one embodiment the method comprises exposing the fluid sample to N-acetyl-L-cysteine before exposing the detection zone. This may be useful if the fluid sample, such as saliva or sputum, is viscous. N-acetyl-L-cysteine is able to disintegrate mucous proteins.

### The porous matrix

The porous matrix can be made from any material capable of absorbing liquid. The porosity of the material can be unidirectional (i.e., with pores or fibers running wholly or predominantly parallel to an axis of the member) or multidirectional (omnidirectional, so that the member has an amorphous sponge-like structure). Porous plastics material, such as polypropylene, polyethylene (preferably of very high molecular weight), polyvinylidene fluoride, ethylene vinyl acetate, acrylonitrile and polytetrafluoroethylene can be used. It can be advantageous to pre-treat the porous matrix with a surface-active agent during manufacture, as this can reduce any inherent hydrophobicity in the member and therefore enhance its ability to take up and deliver a moist sample rapidly and efficiently. The porous matrix can also be made from paper or other cellulosic materials, such as nitrocellulose. Materials that are widely used in the nibs of so-called fiber tipped pens are particularly suitable since such materials can be shaped or extruded in a variety of lengths and shapes. Preferably the porous matrix should be chosen such that the porous material of the matrix can be saturated with aqueous liquid within a matter of seconds. Preferably the material remains robust when moist.

In some embodiments, the porous matrix is selected from the family of nitrocellulose materials. This family has some advantage over conventional synthetic or cellulose materials, such as paper, because it has a natural ability to bind proteins without requiring prior sensitization. The two or more lectins can be applied directly to nitrocellulose and immobilized thereon. No chemical treatment is required which might interfere with the essential specific binding activity of the two or more lectins. Unused binding sites on the nitrocellulose can thereafter be blocked using simple materials, such as polyvinyl alcohol. Moreover, nitrocellulose is generally safe, non-toxic and readily available in a range of pore sizes and this facilitates the selection of a carrier material to suit particularly requirements such as sample flow rate.

Preferably the porous matrix has a pore size of at least one micron. Preferably the porous matrix has a pore size not greater than about 20 microns. In some embodiments, the average pore size of the porous matrix ranges 1-10, 1-20, 1-30, 1-40 or 1-50 microns.

Blocking of unused binding sites in the porous matrix can be achieved by treatment with protein (e.g. bovine serum albumin or milk protein), or with polyvinyl alcohol or ethanolamine, or any combination of these agents, for example. The mobile reagent(s) can then be dispensed onto the dry matrix and will become mobile in the carrier when in the moist state. Between each of these various process steps (sensitization, application of unlabeled reagent, blocking and application of the labeled reagent), the porous matrix should be dried.

### Immobilization of the lectin

The lectin can be applied to the detection zone in a variety of ways. Various "printing" techniques have previously been proposed for application of liquid reagents to porous matrices, e.g., micro-syringes, pens using metered pumps, direct printing and inkjet printing, and any of these techniques can be used in the present context. To facilitate manufacture, the matrix can be treated with the reagents and then subdivided into smaller portions, e.g., small narrow strips each embodying the required reagent-containing zones, to provide a plurality of identical carrier units. In some embodiments, the lectin is permanently immobilized in a detection zone in/on the porous matrix and is therefore not mobile in the moist state e.g. when the probe is soaked with the liquid sample.

### The signal generation means

The signal generation means is typically a label, preferably a visually detectable label. In some embodiments, the lectin is conjugated to the visually detectable label covalently. In some other embodiments, the lectin is conjugated to the visually detectable label non-covalently. The detectable labels are preferably selected such that upon a binding event of the conjugated lectin to the glycan, the visually detectable label has a color intensity level, which has not been visible prior to the binding event, and thus becomes visible thereby making the binding even visibly distinguishable. A particular signal generating means is colloidal gold. In the case of colloidal gold, the visually detectable signal is a result of increase of concentration of the colloidal gold upon binding to the lectin-binding glycan immobilized on the detection zone. Further labels suitable for lateral flow assays have been disclosed in Badir et al. (Trends. Anal. Chem., 82, 2016, pp. 286-306)

The most commonly used detection moieties in lateral flow immunoassays are gold nanoparticles or latex beads. These particles produce a colored readout which requires no development process for visualization. Fluorescent labels, enzymes, other colloidal metals and magnetic particles can also be employed.

In the context of embodiments of the present invention, the term "visible" refer to a visual signal that can be detected by the naked eye (visible light which a human eye can perceive), without the use of additional machinery or processes. In the context of embodiments of the present invention, a visible signal is a change in a color of a certain object or an area thereon, relative to the color that has been characteristic to the object or area prior to the change. A change can also be assessed in comparison to the background of the object or area, and in comparison, to the surrounding of the object or area.

### The lectins

According to the method, the first lectin and the second lectin have similar glycan binding properties. According to a particular embodiment the first lectin and the second lectin are same lectins. According to one embodiment the first lectin and the second lectin is selected those listed in tables 1 and 2. According to another embodiment the first lectin and the second lectin is selected from Aleuria autantia (AAL), Galanthus nivalis (GNA), Hippeastrum hybrid (HHA), Narcissus pseudonarcissus (NPA), Sambucus nigra I (SNA-I), Ulex europaeus I (UEA-I), Urtica dioica (UDA), Allium savitum (ASA), Pisum sativum lectin (PSA), and Phytohemagglutinin-L (PHA-L). According to another embodiment the first lectin and the second lectin is selected from Urtica dioica (UDA), Allium savitum (ASA), Pisum sativum lectin (PSA) and Phytohemagglutinin-L (PHA-L).

According to one embodiment the first lectin and the second lectin is Aleuria autantia (AAL).

According to another embodiment the first lectin and the second lectin is Galanthus nivalis (GNA).

According to another embodiment the first lectin and the second lectin is Hippeastrum hybrid (HHA).

According to another embodiment the first lectin and the second lectin is Narcissus pseudonarcissus (NPA).

According to another embodiment the first lectin and the second lectin is Sambucus nigra I (SNA-I).

According to another embodiment the first lectin and the second lectin is Ulex europaeus I (UEA-I).

According to another embodiment the first lectin and the second lectin is Urtica dioica (UDA).

According to another embodiment the first lectin and the second lectin is Allium savitum (ASA).

According to another embodiment the first lectin and the second lectin is Pisum sativum lectin (PSA).

According to another embodiment the first lectin and the second lectin is Griffithia sp. lectin (GRFT).

According to another embodiment the first lectin and the second lectin is Calystegia sepium lectin (Calsepa).

According to another embodiment the first lectin and the second lectin is Musa acuminata lectin (BanLec).

According to another embodiment the first lectin and the second lectin is Burkholderia cenocepacia lectin (BC2L-A).

According to another embodiment the first lectin and the second lectin is Sambucus Sieboldiana Lectin (SAMB).

According to another embodiment the first lectin and the second lectin is Cicer arietinum lectin (CPA).

According to another embodiment the first lectin and the second lectin is Vigna radiata lectin (VRA).

According to another embodiment the first lectin and the second lectin is Erythrina cristagalli (ECA).

According to another embodiment the first lectin and the second lectin is Human galectin1 lectin (stable form) (Gall).

According to another embodiment the first lectin and the second lectin is Pleurocybella porrigens lectin (PPL).

According to another embodiment the first lectin and the second lectin is Salvia sclarea lectin (SSA).

According to another embodiment the first lectin and the second lectin is Concanavalin A (Con A).

According to another embodiment the first lectin and the second lectin is Phytohemagglutinin-L (PHA-L).

According to a preferable embodiment the first lectin and the second lectin is Urtica dioica (UDA).

Table 1 presents 22 lectins which have been found to be important biomarkers. Data represent patients suffering from Traumatic Brain Injury (TBI), the statistically significant increase marked with (+) compares TBI-patients relative to healthy controls in urine and saliva. Full lectin names together with lectin sources are presented in Table 2.

**Table 1**

| **Lectin abbreviation** | **Specificity** | **Urine** | **Saliva** |
|---|---|---|---|
| AAL | Fucα6GlcNAc | + | |
| ASA | αMan | + | + |
| GNA | αMan | + | + |
| HHA | αMan | + | + |
| NPA | αMan | + | + |
| PSA | αMan, αGlc | + | |
| SNA-I | NANAα(2,6)GalNAc > GalNAc = Lac > GalNANAα(2,6)Gal | + | + |
| UDA | GlcNAc | + | + |
| UEA-I | αFuc | + | |
| PHA-L | Galβ4GlcNAcβ6(GlcNAβ2Manα3)Manα3 | | + |
| GRFT | High mannose | + | + |
| Calsepa | High mannose | + | + |
| BanLec | Mannose, Glucose, branched high-mannose containing α1,3-glycoside bond | + | + |
| BC2 L-A | High mannose | + | + |
| SAMB | NeuAcα2-6Gal/GalNAc | + | |
| CPA | Fetuin | | + |
| VRA | α-Galactose | | + |
| ECA | Galβ4GlcNAc | | + |
| Gal1 | branched LacNAc, Gal | | + |
| PPL | α/βGalNAc | | + |
| SSA | N-Acetylgalactosamine | | + |
| ConA | αMan, αGlc | | + |

**Table 2**

| **Lectin abbreviation** | **Lectin name** | **Source** |
|---|---|---|
| AAL | Aleuria aurantia | Aleuria aurantia mushrooms |
| ASA | Allium sativum | Allium sativum agglutinin (Garlic) |
| GNA | Galanthus nivalis | Galanthus nivalis (Snowdrop) bulbs |
| HHA | Hippeastrum hybrid | Hippeastrum hybrid (Amaryllis) bulbs |
| NPA | Narcissus pseudo narcissus | Narcissus pseudonarcissus (Daffodil) bulbs |
| PSA | Pisum sativum | Pisum sativum (Pea) seeds |
| SNA-I | Sambucus nigra I | Sambucus nigra (Elderberry) bark |
| UDA | Urtica dioica | Urtica dioica (Stinging Nettle) seeds |
| UEA-I | Ulex europaeus I | Ulex europaeus (Furze Gorse) seeds |
| PHA-L | Leucoagglutinin | Phaseolus vulgaris (Red Kidney Bean) seeds |
| GRFT | Griffithia sp. Lectin | Griffithia sp. |
| Calsepa | Calystegia sepium lectin | Calystegia sepium |
| BanLec | Musa acuminata lectin | Musa acuminata |
| BC2 L-A | Burkholderia cenocepacia lectin | Burkholderia cenocepacia |
| SAMB | Sambucus Sieboldiana Lecti | Japanese elderberry |
| CPA | Cicer arietinum lectin | Cicer arietinum (chickpea) |
| VRA | Vigna radiata lectin | mung bean |
| ECA | Erythrina cristagalli | Erythrina cristagalli (Coral Tree) seeds |
| Gal1 | Human galectin1 lectin (stable form) | E. coli expressed human galectin1 (stable form) |
| PPL | Pleurocybella porrigens lectin | Pleurocybella porrigens |
| SSA | Salvia sclarea lectin | Salvia |
| ConA | Concanavalin A | Canavalia ensiformis (Jack Bean) seeds |

The method comprises exposing the detection zone comprising immobilized lectin to the fluid sample. According to one embodiment the fluid sample is applied directly to the detection zone. According to another embodiment the fluid sample is applied to a first part of the porous matrix and allowed to elute through the detection zone towards the second part. If the fluid sample comprises the lectin-binding glycan, it binds to the immobilized lectin adapted to bind the lectin-binding glycan.

The method comprises exposing the detection zone to a conjugate comprising a second lectin and a signal generation means. According to an exemplary embodiment a solution comprising the conjugate is applied on the first part of the porous matrix and allowed to elute through the detection zone towards the second part of the porous matrix. According to another embodiment the solution comprising the conjugate is applied directly on the detection zone.

If the detection zone comprises a lectin-binding glycan bound to the immobilized lectin, the conjugate binds also to the lectin-binding glycan immobilized on the detection zone of the porous matrix via the first lectin.

The method comprises also removing unbound material from the detection zone. According to one embodiment the removing comprises flush the probe, in particular the detection zone with a wash solution which could disturb determining of the lectin-binding ligand. An exemplary wash solution is saline, such as phosphate buffered saline.

The method comprises detecting signal derived from the signal generation means on the detection zone after removal of unbound material. If the detection zone comprises the lectin-binding glycan bound the immobilized lectin, the conjugate binds to the lectin-binding glycan, and the binding is detected based on the signal derived from the signal generating means. When the signal generating means is e.g., colloidal gold, the visible signal is result of increase of colloidal gold concentration on the detection zone.

The method comprises determining level of the lectin-binding glycan in the fluid sample based on the detecting. According to an exemplary embodiment the determining comprises calculating increase of the signal of the signal generation means upon the binding event.

According to a particular embodiment the method comprises also steps of
g) comparing the level of the lectin-binding glycan in the fluid sample to a control level, and
h) determining presence or absence of increased level of the lectin binding glycan in the fluid sample based on the comparing.

According to a particular embodiment the control level is the level of the lectin binding glycan in a body fluid of a subject not suffering from TBI.

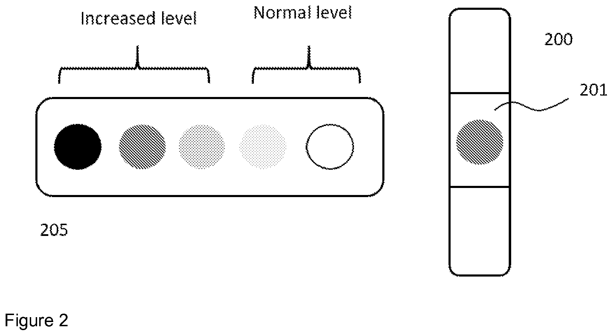

There are different ways to perform steps f) and g). According to one embodiment the steps comprise performing at least two parallel methods: one with a fluid sample with unknown level of lectin-binding glycan indicative to TBI, and another one with body fluid obtainable from a subject not suffering from TBI, or with a solution mimicking a body fluid obtainable from a subject not suffering from TBI. If the level of the lectin-binding glycan is in the fluid sample higher than a predetermined control level e.g., two times higher, the fluid sample has significantly increased level of lectin-binding glycan indicative to TBI. When the signal generating means produces a visually detectable color upon binding to the glycan, the determining can be done by comparing the color obtained to various predetermined colors indicative to different lectin-binding glycan concentrations. The determining can be assisted e.g., with an exemplary test strip 205 as shown in figure 2. According to this embodiment, the porous matrix 200 is allowed to dry and the color on the detection zone 201 is compared with the colors of the test strip.

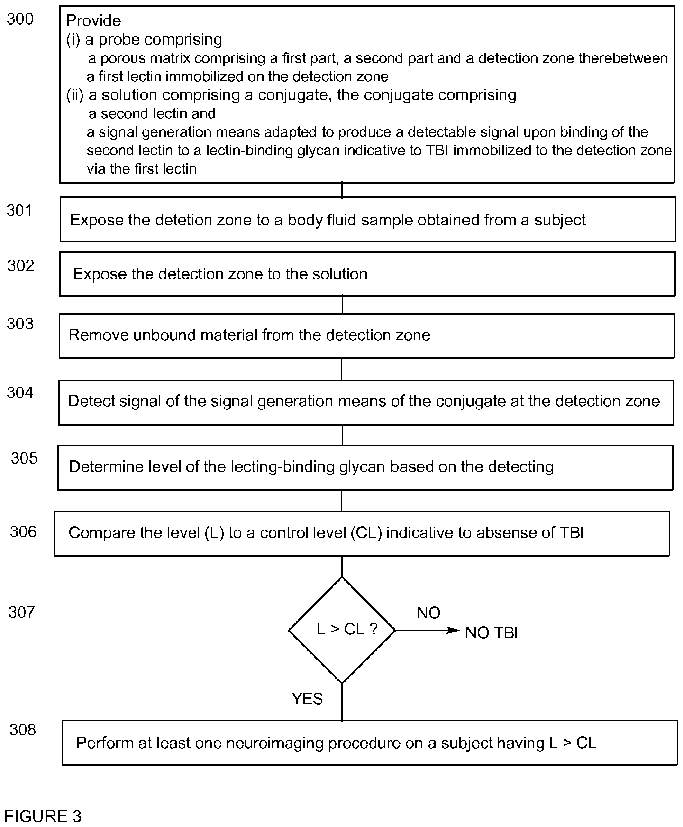

The method of the present invention can also be used for determining traumatic brain injury in a subject. According to this embodiment the steps a) - e) are as disclosed above but steps f) and g) are not optional. The probe, the second lectin and the conjugate may be as disclosed above. The method may also comprise exposing the fluid sample to N-acetyl-L-cysteine before exposing the detection zone. This may be useful if the body fluid sample such as saliva is viscous. The fluid sample is a body fluid sample obtained from a subject suspected to suffer from TBI. An exemplary non-limiting flow chart of the method for determining traumatic brain injury in a subject is shown in figure 3. The method comprises the following actions:
Action 300: Provide
   i. a probe comprising a porous matrix, the porous matrix comprising
      ∘ a first part, a second part and a detection zone therebetween and
      ∘ a first lectin immobilized on the detection zone, and
   ii. a solution comprising a conjugate comprising
      ∘ a second lectin and
      ∘ a signal generation means adapted to produce a detectable signal upon binding of the second lectin to a lectin-binding glycan indicative to TBI immobilized to the detection zone via the first lectin,
      wherein the first lectin and the second lectin have similar glycan binding properties.
Action 301: Expose the detection zone to a body fluid sample obtained from a subject,
Action 302: Expose the detection zone to the solution.
Action 303: Remove unbound material from the detection zone.
Action 304: Detect signal of the signal generation means of the conjugate at the detection zone.
Action 305: Determine level of the lectin-binding glycan in the sample based on the detecting.
Action 306: Compare the determined level of the lectin-binding glycan in the sample to a predetermined control level indicative to absence of TBI.
Action 307: Determine presence or absence of TBI in the subject based on the comparing.

The first lectin and the second lectins are preferably selected from those listed in tables 1 and 2. The first lectin and the second lectin are preferably same lectins.

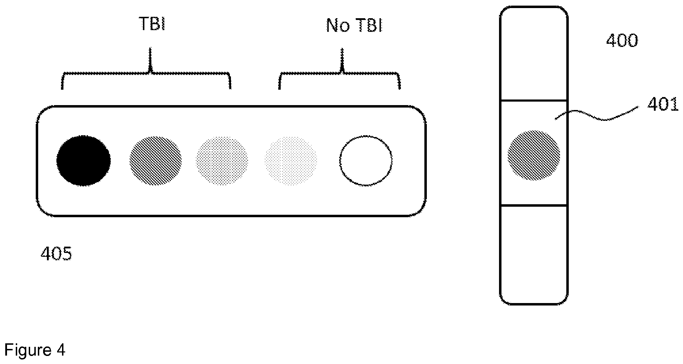

There are different ways to perform Actions 305 and Action 306. According to one embodiment the Actions are done by performing at least two parallel methods: one with a fluid sample with unknown level of lectin-binding glycan indicative to TBI, and another one with body fluid obtainable from a subject not suffering from TBI, or with a solution mimicking a body fluid obtainable from a subject not suffering from TBI. If the level of the lectin-binding glycan in the fluid sample is higher than a predetermined control level e.g., two times higher, the fluid sample has significantly increased level or lectin-binding glycan indicative to TBI. When the signal generating means produces a visually detectable color upon binding to the glycan, the determining can be done by comparing the color obtained to various predetermined colors indicative to different lectin-binding glycan concentrations. The determining can be assisted e.g., with an exemplary test strip 405 shown in figure 4.

According to this embodiment, the porous matrix 400 is allowed to dry and the color on the detection zone 401 is compared with the colors of the test strip.

If the level of the lectin-binding glycan in the body fluid sample is above a control level, the method preferably includes also subjecting the subject to one or more neuroimaging procedures. According to a particular embodiment the method comprises
Action 308: performing at least one neuroimaging procedure selected from a group consisting of x-ray, computerized tomography, and magnetic resonance imaging on a subject having the elevated level of the lectin-binding glycan.

The neuroimaging procedure is preferably performed if the subject has elevated level of the lectin-binding glycan indicative to TBI in the body fluid.

### EXPERIMENTAL

Lectins used were commercially available. Lectin-colloidal gold conjugates were prepared as disclosed in https://www.abbexa.com/lateral-flow-assay
Representative examples of porous matrix materials suitable for the method include paper, nitrocellulose and nylon membranes. Essential features of the material are its ability to bind protein; speed of liquid conduction; and, if necessary, after pre-treatment, its ability to allow the passage of labeled binding reagents therethrough. In embodiments using direct label, it may be desirable for the material to allow flow of particles of size up to a few microns (usually less than 0.5 µm). Examples of flow rates obtained with various materials are presented in Table 3 below, showing the time in minutes to flow through 45 mm of material.

**Table 3**

| Material type | Pore size [µm] | Time [minutes] |
|---|---|---|
| Whatman^{®}'s chromatography paper (Schleicher & Schuell^{®}) unbacked nitrocellulose sheet | | |
| | 3 | 3.40 |
| | 5 | 3.30 |
| | 8 | 3.00 |
| | 12 | 2.20 |
| backed polyester sheet Whatman^{®}'s Nitrocellulose sheet Pall^{®}'s Immunodyne^{®} (nylon) | 8 | 3.40 |
| | 5 | 19.20 |
| | 3 | 4.00 |
| | 5 | 3.20 |

The travel rate of a test procedure will be determined by the flow rate of the material employed and while any of the above materials can be used some will give faster tests than others.

Nitrocellulose is advantageous of requiring no activation and will immobilize proteins strongly by absorption. Immunodyne^{®} is pre-activated and requires no chemical treatment. Papers, such as Whatman^{®} 3MM, require chemical activation with for example carbonyl diimidazole in order to successfully immobilize proteins.

### Example 1

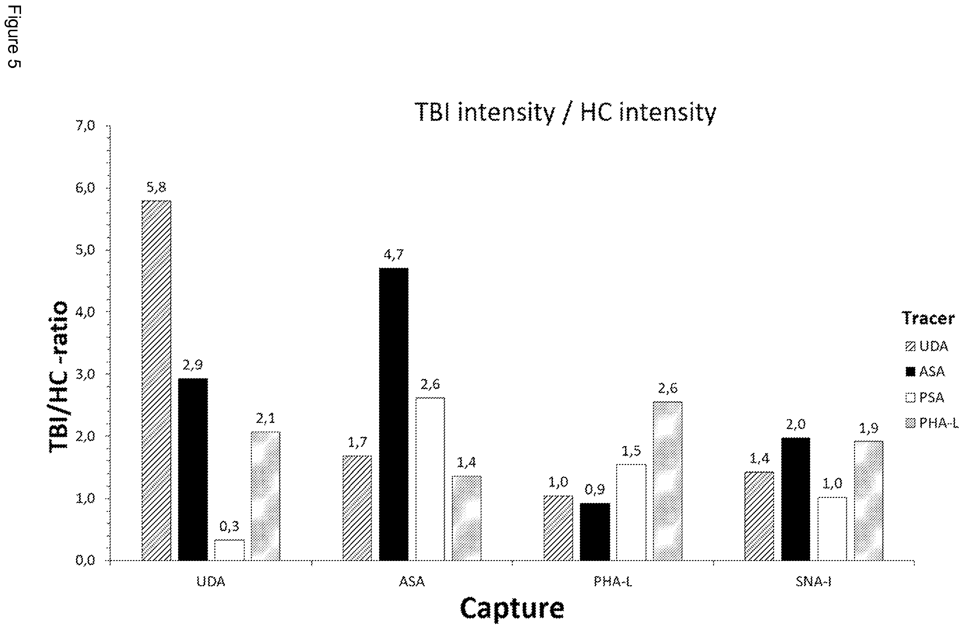

2-3 µL of 2 mg/mL pure lectin in PBS was pipetted on detection zone of a nitrocellulose matrix and allowed to dry for 30 min at 35 °C. Then, 5 µL of sample was pipetted on the lectin. First part of the matrix was soaked to a solution comprising the lectin conjugated with colloidal gold. The solution was allowed to elute from the first part of the matrix towards the second part of the matrix through the detection zone. When the solvent front received second end of the matrix, the matrix was eluted with wash buffer and dried. The procedure was performed using a TBI sample and a healthy control (HC) -sample. The probes were photographed. The pixel intensity of the color on the lectin spots was determined with ImageJ-instrument, a picture analysis tool. Results are shown in figure 5. As shown, the best TBI/HC ratios were obtained when the same lectin was used for tracing and capturing.

### Example 2

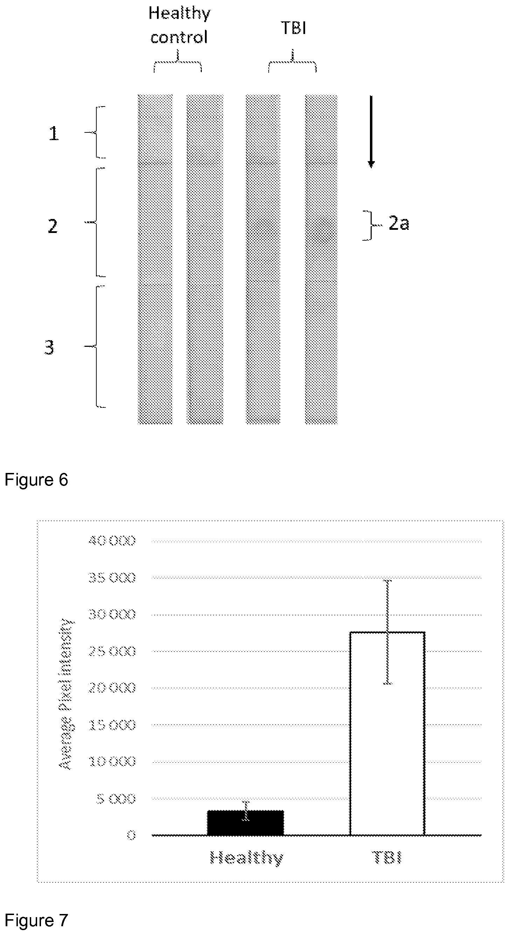

Pure lectin was dissolved in buffer, and 3 µL of the solution was pipetted on the detection zone of the nitrocellulose matrix and allowed to dry at +40 °C for 30 minutes. Human saliva was mixed with phosphate-buffered-saline (PBS), and 5 µL of that dilution was pipetted onto the detection zone over the spot of dried lectin. Wash buffer consisting of PBS and Tween-20 was introduced through the first part of the matrix and the solution was eluted through the detection zone. Colloidal gold-conjugated second lectin was pipetted onto the detection zone and incubated for 5 minutes. Then, 50 µL of wash solution was added to the first part of the matrix and it was allowed to elute through the detection zone to the second part of the matrix. The immobilized first lectin was ASA and the colloidal gold-conjugated second lectin was ASA, i.e., same lectins. Once the wash solution had completely penetrated through the detection zone to the second part of the matrix, the probes were visually inspected and photographed. Results are shown in figure 6. The lectin spot area of the detection zone was analyzed by ImageJ software in order to quantitate the pixel intensity in the reaction area as a numerical value. Average pixel intensities were produced into a graphical presentation shown in figure 7. The average pixel intensity is 8.3 times higher in probes where a sample from a patient with TBI was run, compared to probes where a sample from healthy control subject was run Appearance of the dark spot and increased pixel intensity indicate the formation of a complex consisting of the first lectin, lectin-binding glycan and colloidal gold-conjugated second lectin. The lectin-binding glycan is present in the sample from a patient with TBI but not in healthy control.

### Example 3

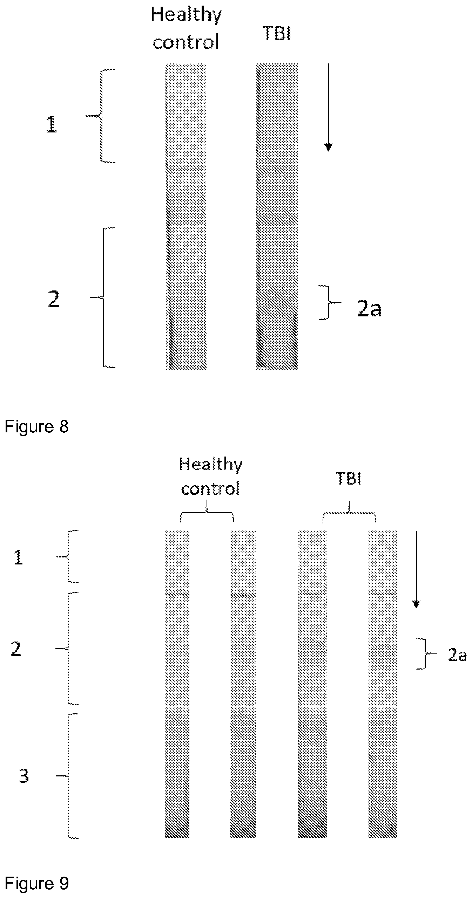

Pure lectin was dissolved in buffer and 2 µL of the lectin solution was pipetted on the detection zone of the probes, and the probes were allowed to dry at +37 °C for 18 hours. Colloidal gold conjugated second lectin was pipetted on the first part of the matrix and allowed to dry at +37 °C for 1 hour. Human saliva was mixed with phosphate-buffered-saline (PBS) containing N-acetylcysteine at final concentration of 50 mM. The first part of the probe was soaked with this sample dilution. The sample fluid that was introduced to the first part of the matrix, dissolved the dried colloidal gold-conjugated second lectin from the matrix, and the mixture was allowed to elute to the detection zone. Once the liquid had migrated through the detection zone to the second part of the probe, the first part of the matrix was soaked with wash buffer containing PBS/Tween-20. The immobilized first lectin was SNA-I and the colloidal gold-conjugated second lectin was SNA-I, i.e., same lectins. The probes were photographed once all the wash buffer had migrated through the detection zone. Results are shown in figure 8. A sample from a patient with TBI generated a dark spot indicating the presence of the lectin-binding glycan in the sample.

### Example 4

Pure lectin was dissolved in buffer and 2 µL of the lectin solution was pipetted on the detection zone of a nitrocellulose matrix and allowed to dry for 18 hours at +37 °C. Then, 20 µL of undiluted urine sample was pipetted to the first part of the matrix and allowed to elute from the first part of the matrix towards the second part through the detection zone for 5 minutes. A volume of 10 µL of colloidal gold-conjugated second lectin was pipetted to the first part of the matrix and allowed to elute from the first part towards the second part through the detection zone. The first part of the matrix was soaked with 50 µL of PBS/Tween-20 and the wash solution was allowed to elute through the detection zone. The result was read in 10 minutes. The experiment was performed with a sample from a TBI-patient and a sample from non-injured healthy control subject (HC), each on two parallel probes. The immobilized first lectin was SNA-I and the colloidal gold-conjugated second lectin was SNA-I, i.e., same lectins. The probes were inspected visually and photographed. Results are shown in figure 9. A dark spot appeared on the probes with TBI-samples, indicating formation of complex consisting of first lectin, lectin-binding glycan from the TBI-sample and second lectin conjugated to colloidal gold label. The detection zone of the probes with HC-sample remained clear which indicates that the fluid sample from healthy control did not contain such lectin-binding glycan which would have contributed to complex formation and color development.

The specific examples provided in the description given above should not be construed as limiting the scope and/or the applicability of the appended claims.

## Claims

1. A method for determining a lectin-binding glycan indicative to traumatic brain injury (TBI) in a fluid sample, the method comprising
a) providing
i. a probe comprising a porous matrix, the porous matrix comprising
∘ a first part, a second part and a detection zone therebetween and
∘ a first lectin adapted to bind the lectin-binding glycan and being immobilized to the detection zone, and
ii. a conjugate comprising
∘ a second lectin adapted to bind the lectin-binding glycan and
∘ a signal generation means adapted to produce a detectable signal upon binding of the second lectin of the conjugate to the lectin-binding glycan immobilized to the detection zone via the first lectin,
wherein the first lectin and the second lectin are same lectins and are selected from a group consisting of Urtica dioica (UDA), Allium savitum (ASA) and Phytohemagglutinin-L (PHA-L),
b) exposing the detection zone to the fluid sample,
c) exposing the detection zone to the conjugate,
d) detecting signal derived from the signal generation means at the detection zone,
e) determining level of the lectin-binding glycan in the fluid sample based on the detecting, and
f) comparing the level of the lectin-binding glycan in the fluid sample to a control level indicative to a level of the lectin-binding glycan in a body fluid of a subject not suffering from TBI, wherein said lectin-binding glucan is indicative to TBI when its level in the fluid sample is at least two times higher than the control level.

2. The method according to claim 1 or 2 comprising removing unbound material from the detection zone before the detecting of step d).

3. The method according to claim 1 or 2 wherein the exposing of step b) comprises applying the fluid sample onto to the detection zone, or
applying the fluid sample onto the first part of the porous matrix and allowing the fluid sample to elute through the detection zone towards the second part.

4. The method according to any one of claims 1-3 wherein the exposing of step c) comprises applying a solution comprising the conjugate to the first part of the porous matrix and allowing the solution to elute through the detection zone towards the second part.

5. The method according to any one of claims 1-4 wherein the fluid is body fluid selected from saliva, urine, serum and blood plasma, tears, cerebrospinal fluid, oral secretions, respiratory secretions, preferably from saliva and urine.

6. The method according to claim 5 wherein the body fluid is saliva, and wherein the method comprises exposing the fluid sample to N-acetyl-L-cysteine before step b).

7. The method according to any one of claims 1-6 wherein the first lectin and the second lectin is UDA.

8. The method according to any one of claims 1-7 wherein the signal generation means is adapted to produce visually detectable color upon the binding.

9. The method according to any one of claims 1-8 wherein the signal generation means comprises colloidal gold.

10. A method for determining traumatic brain injury (TBI) in a subject, the method comprising
a) providing
i. a probe comprising a porous matrix, the porous matrix comprising
∘ a first part, a second part and a detection zone therebetween and
∘ a first lectin adapted to bind a lectin-binding glycan indicative to TBI, and being immobilized to the detection zone, and
ii. a conjugate comprising
∘ a second lectin adapted to bind the lectin-binding glycan indicative to TBI and
∘ a signal generation means adapted to produce a detectable signal upon binding of the second lectin to the lectin-binding glycan immobilized to the detection zone via the first lectin,
wherein the first lectin and the second lectin are same lectins and are selected from a group consisting of Urtica dioica (UDA), Allium savitum (ASA) and Phytohemagglutinin-L (PHA-L),
b) exposing the detection zone to a body fluid sample obtained from the subject,
c) exposing the detection zone to the conjugate,
d) detecting signal derived from the signal generation means on the detection zone,
e) determining level of the lectin-binding glycan in the body fluid sample based on the detecting,
f) comparing the level of the lectin-binding glycan in the body fluid sample to a control level indicative to a level of the lectin-binding glycan in body fluid of a subject not suffering from TBI, wherein said lectin-biding glycan is indicative to TBI when its level in the fluid sample is at least two times higher than the control level and
g) determining TBI in the subject based on the comparing.

11. The method according to claim 10 comprising removing unbound material from the probe before the detecting of step d).

12. The method according to claim 10 or 11 wherein the body fluid is selected from saliva, urine, serum and blood plasma, tears, cerebrospinal fluid, oral secretions, respiratory secretions.

13. The method according to claim 12 wherein the body fluid is saliva, and the method comprises exposing the fluid sample to N-acetyl-L-cysteine before step b).

14. The method according to any one of claims 10-13 wherein the first lectin and the second lectin is UDA.

15. The method according to any one of claims 10-14 comprising
h) performing at least one neuroimaging procedure selected from a group consisting of x-ray, computerized tomography, and magnetic resonance imaging on the subject and determining the presence of TBI in the subject based on the elevated level of the lectin-binding glycan and the neuroimaging.

## Patentansprüche

1. Verfahren zur Bestimmung eines lektinbindenden Glycans zur Anzeige einer traumatischen Hirnverletzung (TBI - *Traumatic Brain Injury)* in einer Fluidprobe, wobei das Verfahren Folgendes umfasst:
a) Bereitstellen
i. einer Sonde, welche eine poröse Matrix umfasst, wobei die poröse Matrix Folgendes umfasst:
∘ einen ersten Teil, einen zweiten Teil und eine Nachweiszone dazwischen, und
∘ ein erstes Lektin, das dazu geeignet ist, das lektinbindende Glycan zu binden und an der der Nachweiszone immobilisiert zu werden, und
ii. eines Konjugates, welches Folgendes umfasst:
∘ ein zweites Lektin, das zum Binden des lektinbindenden Glycans geeignet ist, und
∘ ein Signalerzeugungsmittel, das beim Binden des zweiten Lektins des Konjugates an das lektinbindende Glycan, das über das erste Lektin an der Nachweiszone immobilisiert ist, zum Erzeugen eines nachweisbaren Signals geeignet ist,
wobei das erste Lektin und das zweite Lektin die gleichen Lektine sind und ausgewählt sind aus einer Gruppe bestehend aus Urtica dioica (UDA), Allium sativum (ASA) und Phytohämagglutinin-L (PHA-L),
b) Aussetzen der Nachweiszone gegenüber der Fluidprobe,
c) Aussetzen der Nachweiszone gegenüber dem Konjugat,
d) Nachweisen eines Signals, das von dem Signalerzeugungsmittel abgeleitet ist, in der Nachweiszone,
e) Bestimmen eines Levels des lektinbindenden Glycans in der Fluidprobe basierend auf dem Nachweisen, und
f) Vergleichen des Levels des lektinbindenden Glycans in der Fluidprobe mit einem Kontrolllevel zur Anzeige eines Levels des lektinbindenden Glycans in einem Körperfluid eines Subjektes, das nicht an einer TBI leidet, wobei das lektinbindende Glycan auf eine TBI hindeutet, wenn sein Level in der Fluidprobe mindestens zwei Mal höher als das Kontrolllevel ist.

2. Verfahren nach Anspruch 1 oder 2, welches das Entfernen von ungebundenem Material aus der Nachweiszone vor dem Nachweisen von Schritt d) umfasst.

3. Verfahren nach Anspruch 1 oder 2, wobei das Aussetzen von Schritt b) das Auftragen der Fluidprobe auf die Nachweiszone oder das Auftragen der Fluidprobe auf den ersten Teil der porösen Matrix und das Gestatten, dass die Fluidprobe durch die Nachweiszone hin zum zweiten Teil eluiert, umfasst.

4. Verfahren nach einem der Ansprüche 1-3, wobei das Aussetzen von Schritt c) das Auftragen einer Lösung, welche das Konjugat umfasst, auf den ersten Teil der porösen Matrix und das Gestatten, dass die Lösung durch die Nachweiszone hin zum zweiten Teil eluiert, umfasst.

5. Verfahren nach einem der Ansprüche 1-4, wobei das Fluid ein Körperfluid ausgewählt aus Speichel, Urin, Serum und Blutplasma, Tränen, Rückenmarksflüssigkeit, oralen Sekreten oder respiratorischen Sekreten ist, vorzugsweise aus Speichel und Urin.

6. Verfahren nach Anspruch 5, wobei das Körperfluid Speichel ist und wobei das Verfahren das Aussetzen der Fluidprobe gegenüber N-Acetyl-L-Cystein vor Schritt b) umfasst.

7. Verfahren nach einem der Ansprüche 1-6, wobei das erste Lektin und das zweite Lektin UDA sind.

8. Verfahren nach einem der Ansprüche 1-7, wobei das Signalerzeugungsmittel bei der Bindung zum Erzeugen von visuell nachweisbarer Farbe geeignet ist.

9. Verfahren nach einem der Ansprüche 1-8, wobei das Signalerzeugungsmittel kolloidales Gold umfasst.

10. Verfahren zur Bestimmung einer traumatischen Hirnverletzung (TBI - *Traumatic Brain Injury)* bei einem Subjekt, wobei das Verfahren Folgendes umfasst:
a) Bereitstellen
i. einer Sonde, welche eine poröse Matrix umfasst, wobei die poröse Matrix Folgendes umfasst:
∘ einen ersten Teil, einen zweiten Teil und eine Nachweiszone dazwischen, und
∘ ein erstes Lektin, das dazu geeignet ist, ein lektinbindendes Glycan, das auf eine TBI hindeutet, zu binden und an der Nachweiszone immobilisiert zu werden, und
ii. eines Konjugates, welches Folgendes umfasst:
∘ ein zweites Lektin, das zum Binden des lektinbindenden Glycans, das auf eine TBI hindeutet, geeignet ist, und
∘ ein Signalerzeugungsmittel, das beim Binden des zweiten Lektins an das lektinbindende Glycan, das über das erste Lektin an der Nachweiszone immobilisiert ist, zum Erzeugen eines nachweisbaren Signals geeignet ist, wobei das erste Lektin und das zweite Lektin die gleichen Lektine sind und ausgewählt sind aus einer Gruppe bestehend aus Urtica dioica (UDA), Allium sativum (ASA) und Phytohämagglutinin-L (PHA-L),
b) Aussetzen der Nachweiszone gegenüber einer Körperfluidprobe, die von dem Subjekt erhalten wird,
c) Aussetzen der Nachweiszone gegenüber dem Konjugat,
d) Nachweisen eines Signals, das von dem Signalerzeugungsmittel abgeleitet ist, in der Nachweiszone,
e) Bestimmen eines Levels des lektinbindenden Glycans in der Körperfluidprobe basierend auf dem Nachweisen,
f) Vergleichen des Levels des lektinbindenden Glycans in der Körperfluidprobe mit einem Kontrolllevel zur Anzeige eines Levels des lektinbindenden Glycans in einem Körperfluid eines Subjektes, das nicht an einer TBI leidet, wobei das lektinbindende Glycan auf eine TBI hindeutet, wenn sein Level in der Fluidprobe mindestens zwei Mal höher als das Kontrolllevel ist, und
g) Bestimmen einer TBI bei dem Subjekt basierend auf dem Vergleichen.

11. Verfahren nach Anspruch 10, welches das Entfernen von ungebundenem Material von der Sonde vor dem Nachweisen von Schritt d) umfasst.

12. Verfahren nach Anspruch 10 oder 11, wobei das Körperfluid aus Speichel, Urin, Serum und Blutplasma, Tränen, Rückenmarksflüssigkeit, oralen Sekreten oder respiratorischen Sekreten ausgewählt ist.

13. Verfahren nach Anspruch 12, wobei das Körperfluid Speichel ist und wobei das Verfahren das Aussetzen der Fluidprobe gegenüber N-Acetyl-L-Cystein vor Schritt b) umfasst.

14. Verfahren nach einem der Ansprüche 10-13, wobei das erste Lektin und das zweite Lektin UDA sind.

15. Verfahren nach einem der Ansprüche 10-14, welches Folgendes umfasst:
h) Durchführen von mindestens einem Neurobildgebungsvorgang ausgewählt aus einer Gruppe bestehend aus Röntgen, Computertomografie und Magnetresonanztomografie an dem Subjekt und Bestimmen des Vorhandenseins einer TBI bei dem Subjekt basierend auf dem erhöhten Level des lektinbindenden Glycans und der Neurobildgebung.

## Revendications

1. Procédé de détermination d'un glycane se liant à une lectine indicateur d'une lésion cérébrale traumatique (TBI) dans un échantillon de fluide, le procédé comprenant les étapes consistant à
a) fournir
i. une sonde comprenant une matrice poreuse, la matrice poreuse comprenant
• une première partie, une seconde partie et une zone de détection entre elles et
• une première lectine adaptée pour se lier au glycane se liant à une lectine et étant immobilisée à la zone de détection et
ii. un conjugué comprenant
• une seconde lectine adaptée pour se lier au glycane se liant à une lectine et
• un moyen de production de signal adapté pour produire un signal détectable après liaison de la seconde lectine du conjugué au glycane se liant à une lectine immobilisé à la zone de détection via la première lectine,
la première lectine et la seconde lectine étant les mêmes lectines et étant choisies dans un groupe constitué par Urtica dioica (UDA), Allium savitum (ASA) et la phytohémagglutinine-L (PHA-L),
b) exposer la zone de détection à l'échantillon de fluide,
c) exposer la zone de détection au conjugué,
d) détecter le signal dérivé du moyen de production de signal à la zone de détection,
e) déterminer le niveau du glycane se liant à une lectine dans l'échantillon de fluide sur la base de la détection et
f) comparer le niveau du glycane se liant à une lectine dans l'échantillon de fluide à un niveau témoin indicateur d'un niveau du glycane se liant à une lectine dans un échantillon de fluide d'un sujet ne souffrant pas de TBI, ledit glycane se liant à une lectine étant indicateur de TBI quand son niveau dans l'échantillon de fluide est au moins deux fois supérieur au niveau témoin.

2. Procédé selon la revendication 1 ou 2 comprenant l'enlèvement du matériau non lié de la zone de détection avant la détection de l'étape d).

3. Procédé selon la revendication 1 ou 2 dans lequel l'exposition de l'étape b) comprend l'application de l'échantillon de fluide sur la zone de détection ou l'application de l'échantillon de fluide sur la première partie de la matrice poreuse et permettant à l'échantillon de fluide d'éluer à travers la zone de détection vers la seconde partie.

4. Procédé selon l'une quelconque des revendications 1 à 3 dans lequel l'exposition de l'étape c) comprend l'application d'une solution comprenant le conjugué à la première partie de la matrice poreuse et permettant à la solution d'éluer à travers la zone de détection vers la seconde partie.

5. Procédé selon l'une quelconque des revendications 1 à 4 dans lequel le fluide est un fluide corporel choisi parmi la salive, l'urine, le sérum et le plasma sanguin, les larmes, le fluide cérébrospinal, les sécrétions orales, les sécrétions respiratoires, de préférence parmi la salive et l'urine.

6. Procédé selon la revendication 5 dans lequel le fluide corporel est la salive et le procédé comprenant l'exposition de l'échantillon de fluide à la N-acétyl-L-cystéine avant l'étape b).

7. Procédé selon l'une quelconque des revendications 1 à 6 dans lequel la première lectine et la seconde lectine sont l'UDA.

8. Procédé selon l'une quelconque des revendications 1 à 7 dans lequel le moyen de production de signal est adapté pour produire une couleur visuellement détectable après la liaison.

9. Procédé selon l'une quelconque des revendications 1 à 8 dans lequel le moyen de production de signal comprend de l'or colloïdal.

10. Procédé de détermination de lésion cérébrale traumatique (TBI) chez un sujet, le procédé comprenant les étapes consistant à
a) fournir
i. une sonde comprenant une matrice poreuse, la matrice poreuse comprenant
• une première partie, une seconde partie et une zone de détection entre elles et
• une première lectine adaptée pour se lier à un glycane se liant à une lectine indicateur de TBI et étant immobilisée à la zone de détection et
ii. un conjugué comprenant
• une seconde lectine adaptée pour se lier au glycane se liant à une lectine indicateur de TBI et
• un moyen de production de signal adapté pour produire un signal détectable après la liaison de la seconde lectine au glycane se liant à une lectine immobilisée à la zone de détection via la première lectine,
la première lectine et la seconde lectine étant les mêmes lectines et étant choisies dans un groupe constitué par Urtica dioica (UDA), Allium savitum (ASA) et la phytohémagglutinine-L (PHA-L),
b) exposer la zone de détection à un échantillon de fluide corporel obtenu du sujet,
c) exposer la zone de détection au conjugué,
d) détecter le signal dérivé du moyen de production de signal sur la zone de détection,
e) déterminer le niveau du glycane se liant à une lectine dans l'échantillon de fluide corporel sur la base de la détection,
f) comparer le niveau du glycane se liant à une lectine dans l'échantillon de fluide corporel à un niveau témoin indicateur d'un niveau du glycane se liant à une lectine dans le fluide corporel d'un sujet ne souffrant pas de TBI, ledit glycane se liant à une lectine étant indicateur de TBI quand son niveau dans l'échantillon de fluide est au moins deux fois supérieur au niveau témoin et
g) déterminer une TBI chez le sujet sur la base de la comparaison.

11. Procédé selon la revendication 10 comprenant l'enlèvement du matériau non lié de la sonde avant la détection de l'étape d).

12. Procédé selon la revendication 10 ou 11 dans lequel le fluide corporel est choisi parmi la salive, l'urine, le sérum et le plasma sanguin, les larmes, le fluide cérébrospinal, les sécrétions orales, les sécrétions respiratoires.

13. Procédé selon la revendication 12 dans lequel le fluide corporel est la salive et le procédé comprend l'exposition de l'échantillon de fluide à la N-acétyl-L-cystéine avant l'étape b).

14. Procédé selon l'une quelconque des revendications 10 à 13 dans lequel la première lectine et la seconde lectine sont l'UDA.

15. Procédé selon les revendications 10 à 14 comprenant
h) d'effectuer au moins une procédure de neuroimagerie choisie dans un groupe constitué par les rayons X, la tomographie informatique et l'imagerie par résonance magnétique sur le sujet et de déterminer la présence de TBI chez le sujet sur la base du niveau élevé du glycane se liant à une lectine et de la neuroimagerie.
